# EUROPEAN PATENT APPLICATION

(11) **EP 2 985 038 A1**
(43) Date of publication of application: **17.02.2016**
(21) Application number: 14180675.2
(22) Date of filing: 12.08.2014
(51) Int. Cl.: A61K 47/22, A61K 9/19, A61K 31/4184

(54) **Lyophilized API preparation**

(71) Applicant: Azad Pharma AG, 3125 Toffen (CH)
(72) Inventor: Ondracek, Jan, 8207 Schaffhausen (CH); Baronian, Mihran, 3125 Toffen (CH)
(74) Representative: Weickmann & Weickmann

(57) **Abstract**

The present invention relates to lyophilized preparations comprising an active pharmaceutical ingredient (API) for pharmaceutical use as well as methods of preparing same.

## Description

The present invention relates to lyophilized preparations comprising an active pharmaceutical ingredient (API) for pharmaceutical use as well as methods of preparing same.

Because of their high reactivity in aqueous solutions, some APIs are difficult to formulate as pharmaceuticals and are often supplied in a lyophilized dry form that requires reconstitution in a liquid medium (e.g. water) by a skilled person prior to administration. In aqueous solution the APIs may degrade by hydrolysis or other degradation processes during the manufacture, lyophilization process and/or after reconstitution. The time period of API exposure to water during lyophilization and after reconstitution increases impurity formation and consequently results in a potential loss of efficacy. Accordingly, the lyophilization and reconstitution process is to be conducted in a gentle manner and the reconstituted product should be administered to a patient as soon as possible after reconstitution.

APIs which are particularly affected by a hydrolytic degradation are, for example, nitrogen mustards such as Bendamustine (4-[5-[Bis(2-chloroethyl)amino]-1-methylbenzimidazol-2-yl]butanoic acid) which corresponds to the following formula:

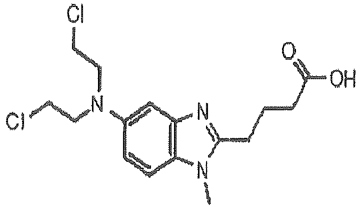

Bendamustine hydrochloride was initially synthesized in 1963 by W. Ozegowski and D. Krebs. The molecule was originally named IMET 3393, an active agent with both alkylating and antimetabolite properties.

From 1971 to 1990, it was available only in the German Democratic Republic under the trademark Cytostasan^{®} which was provided as a lyophilized powder which had to be reconstituted for parenteral administration. Provision of a lyophilisate became necessary due to degradation of Bendamustine in aqueous solutions, particularly hydrolysis of the bis-2-chloroethylamino-group of Bendamustine (B. Maas et al., 1994, Pharmazie 49: 775-777). Bendamustine has been used to treat chronic lymphocytic leukemia, non-Hodgkin's lymphoma and multiple myeloma, but never studied systematically in patients until the 1990s when German investigators demonstrated its clinical activity in a number of malignancies.

The current lyophilized formulations of Bendamustine, Ribomustine^{®} (Europe) and Treanda^{®} (US) contain Bendamustine hydrochloride and mannitol in a sterile lyophilized powder form in a vial for intravenous use following reconstitution and dilution. The current lyophilized preparations contain degradation products that may occur during manufacturing of the drug substance and/or during the lyophilization process to make the finished drug product.

A lot of efforts have been made to optimize the lyophilization process for reducing the impurity content in the lyophilized product.

WO 2006/076620 discloses pharmaceutical compositions of Bendamustine containing not more than about 0.9 % of 5-[(2-chloroethyl)-(2-hydroxy-ethyl)-amino]-1-methyl-1H-benzimidazol-2-yl)-butyric acid (HP1; Monohydroxybendamustine), present at time 0 after reconstitution of a lyophilized preparation. The lyophilized preparation is produced by dissolving Bendamustine in a stabilizing concentration of an alcohol solvent to form a pre-lyohilization solution and subsequent lyophilization of the pre-lyophilization solution. Suitable alcohols used in this process are, e.g., methanol, ethanol, propanol, isopropanol, butanol and tert-butanol.

WO 2009/120386 discloses a lyophilized composition comprising specific crystalline forms of Bendamustine hydrochloride. The lyophilization process is carried out using mixtures of water and organic solvents such as ethanol, n-propanol, isopropanol, n-butanol, tert-butanol or a mixture thereof.

US 2013/0041003 discloses a pharmaceutical composition comprising Bendamustine or Bendamustine hydrochloride, mannitol, water and a solvent that is ethanol, n-propanol, n-butanol, iso-propanol, methanol, ethyl acetate, dimethyl carbonate, acetonitrile, dichloromethane, methyl ethyl ketone, methyl isobutyl ketone, acetone, 1-pentanol, methyl acetate, carbon tetrachloride, dimethyl sulfoxide, hexafluoroacetone, chlorobutanol, dimethyl sulfone, acetic acid, cyclohexane, or a combination thereof.

US 2013/0123316 discloses a stable lyophilized preparation comprising Bendamustine hydrochloride, mannitol and tert-butyl alcohol, wherein the ratio by weight of Bendamustine hydrochloride to mannitol is 15 : 25.5.

Lyophilized cyclophosphamide preparations and their manufacture are described in US 5,418,223, US 5,413,995, US 5,268,368, US 5,227,374, US 5,130,305, US 4,659,699, US 4,537,883 and US 5,066,647.

From the above it becomes clear that there is still a need for lyophilized formulations of API that have a reduced amount of impurities in the lyophilized preparation.

Thus, in one aspect, the present invention provides a preparation comprising:
(i) at least one active pharmaceutical ingredient (API), a salt, hydrate, solvate or prodrug thereof, and
(ii) at least one bulking agent selected from monosaccharides, disaccharides, oligosaccharides, polysaccharides and sugar alcohols,
(iii) at least one organic solvent having a vapor pressure of 1.0 to 41 mbar at 20°C, and
(iv) water.

The active pharmaceutical ingredient (API) of the preparation of the invention is preferably not stable in the presence of water or humidity, i.e. may undergo a structural change due to degradation processes, particularly hydrolysis. In a preferred embodiment the term "not stable in the presence of water or humidity" means that at least 0.2 wt.-%, preferably at least 0.5 wt.-%, more preferably at least 1 wt.-% of the API starting material is degraded (structurally changed) when stored in distilled water at 20°C for 12 hours.

In a preferred embodiment, the API according to the invention is selected from a nitrogen mustard such as Bendamustine, Busulfan, Melphalan, Uramustine, Ifosfamide, Chlorambucil and Cyclophosphamide; cytostatic agents such as Fludarabine, Doxorubicine, Pemetrexed and Dacarbazine; and opioids such as Remifentanil. In a preferred embodiment the API of the present invention is Bendamustine.

The preparation according to the present invention may also comprise pharmaceutically acceptable salts, hydrates, solvates or prodrugs of the API. The term *"pharmaceutically acceptable salt"* as used herein describes any pharmaceutically acceptable salt that administered to a patient (directly or indirectly) provides API. For example, pharmaceutically acceptable salts are synthesized from corresponding acids or bases. Examples of acids which may be useful for the salt formation of the pharmaceutically acceptable salts of API include inorganic acids such as hydrochloric acid, hydrobromic acid, hydroiodic acid, sulfuric acid, nitric acid and phosphoric acid, and organic acids such as acetic acid, malic acid, fumaric acid, citric acid, oxalic acid, succinic acid, tartaric acid, lactic acid, methylsulfonic acid and p-toluene sulfonic acid. Examples of bases used in the formation of API salts may be either inorganic or organic bases such as ammonia, alkaline (e.g. lithium, sodium, potassium, etc.) hydroxide, earth alkaline (e.g. magnesium, aluminum, calcium, etc.) hydroxide, C₁-C₆ alkylamine such as methylamine or ethylamine, C₁-C₆ alkyl diamine such as ethylene diamine, ethanolamine, N,N-dialkyleneethanol amine, triethanol amine, glucamine and amino acids. Particularly preferred are acid addition salts - such as hydrochloride, hydrobromide and hydroiodide salts, wherein the hydrochloride salt is the most preferred pharmaceutically acceptable salt. The pharmaceutically acceptable salts are produced by conventional techniques well-known in the art.

The expression *"pharmaceutically acceptable solvate thereof"* describes any pharmaceutically acceptable solvate that administered to a patient (directly or indirectly) provides API. Preferably the solvate is a solvate with an alcohol such as methanol, ethanol, propanol or isopropanol, a solvate with an ester such as ethyl acetate, and ether such as methyl ether, ethyl ether or tetrahydrofuran or with dimethylformamide. In a preferred embodiment the solvate is an alcohol such as an ethanol solvate.

In another aspect, the API of the present invention may be provided as an API prodrug which is metabolized to the respective API when passing the body of the subject to be treated. Preferred prodrugs of the invention are pharmaceutically acceptable esters of the API such as esters with alkyl alcohols and saccharide alcohols. Examples of the alkyl alcohols are C₁-C₆ alkyl alcohols such as methanol, ethanol, propanol, isopropanol, butanol and tert-butanol. Examples of the saccharide alcohols are mannitol, maltitol, sorbitol, erythritol, glycol, glycerol, arabitol, xylitol and lactitol.

In a preferred embodiment of the present invention, the API is Bendamustine, a salt, hydrate, solvate or a prodrug thereof. Particularly preferred is a Bendamustine salt (hydrate) such as Bendamustine hydrochloride or its hydrate. Preferred Bendamustine prodrugs are Bendamustine ethyl ester, Bendamustine isopropyl ester, Bendamustine mannitol ester and Bendamustine sorbitol ester, in particular Bendamustine ethyl ester.

The preparation of the invention further comprises at least one bulking agent selected from monosaccharides such as glucose, fructose, dextrose and galactose; disaccharides such as sucrose, lactulose, lactose, maltose, or trehalose; oligosaccharides such as galactooligosaccharide; polysaccharides such as cellulose; and sugar alcohols such as erythritol, xylitol, mannitol, maltitol, sorbitol, or lactitol. In a preferred embodiment, the bulking agent is selected from monosaccharides, disaccharides and sugar alcohols. In another preferred embodiment, the bulking agent is selected from mannitol, maltitol, erythritol, xylitol, lactose, sucrose, glucose, sorbitol, maltose, trehalose, lactitol and dextrose, more preferably mannitol.

The preparation of the invention further comprises at least one organic solvent having a vapor pressure of about 1.0 to 41 mbar at 20°C. It has been found that within this range of vapor pressure, the lyophilization process can be carried out at reasonable efforts in view of the lyophilization equipment and process and simultaneously results in lyophilisates having reduced amounts of impurities.

Suitable organic solvents according to the present invention are preferably pharmaceutically acceptable and can be used in medical applications without causing any side effects. Preferably the organic solvent is selected from ethyl lactate, dioxane, particularly 1,4-dioxane, isopropylidene glycerol (solketal), acetic acid, acetyl acetone, 1-butanol, 1-propanol, N,N-dimethyl acetamide, cyclohexanone and toluene. In a preferred embodiment, the organic solvent is selected from ethyl lactate, dioxane, solketal and mixtures thereof. In another embodiment the organic solvent is selected from ethyl lactate, dioxane, particularly 1,4-dioxane, acetic acid, acetyl acetone, 1-butanol, 1-propanol, N,N-dimethyl acetamide, cyclohexanone and toluene. In a preferred embodiment, the organic solvent is selected from ethyl lactate and dioxane, particularly ethyl lactate and 1,4-dioxane, and most particularly ethyl lactate.

In a preferred embodiment, the preparation of the present invention comprises 1 - 80 % by weight, preferably 20-60 % by weight, even more preferably 30-50 % by weight of at least one organic solvent based on the total amount of the preparation.

The preparation may further comprise 19.3 - 99.3 % by weight, preferably 20-95 % by weight, more preferably 40-75 % by weight, even more preferably 40-70 % by weight of water based on the total amount of the preparation.

In a preferred embodiment, the at least one API may be present in an amount of 0.01-10 % by weight, preferably 0.2-10 % by weight, more preferably 0.5-5 % by weight, even more preferably 0.5-4 % by weight based on the total amount of the preparation.

The at least one bulking agent may be present in the preparation in an amount of 0.5-15 % by weight, preferably 0.5-10 % by weight, more preferably 1-7 % by weight based on the total amount of the preparation.

The preparation of the present invention may further comprise at least one pharmaceutically acceptable excipient such as stabilizers, buffers, solubilizers, anti-bacterial agents such as paraben, chlorobutanol, phenol, ascorbic acid and dispersion medium such as polyose (e.g. glycerol, propylenglycol, liquid polyethylene glycol), fats (e.g. vegetable oils, non-toxic glycerol esters) and mixtures thereof.

The lyophilized preparation may further comprise a further pharmaceutical active agent such as, for example, another antineoplastic agent.

In one aspect of the invention, the preparation of the invention comprises:
(i) at least one Bendamustine, a salt, hydrate, solvate or a prodrug thereof, in particular Bendamustine hydrochloride or Bendamustine hydrochloride hydrate,
(ii) at least one bulking agent selected from mannitol,
(iii) at least one organic solvent having a vapor pressure of 1.0 - 41 mbar at 20°C, particularly ethyl lactate and/or 1,4-dioxane, more preferably ethyl lactate, and
(iv) water.

In this specific embodiment, the preparation may contain a concentration of about 22 mg/ml of Bendamustine hydrochloride, about 37 mg/ml of mannitol, about 5-50 % of a mixture of 1,4-dioxane and water or a mixture of ethyl lactate and water.

The preparation of the invention preferably forms a solution at room temperature (e.g. 20°C), i.e. any components of the preparation are molecularly dissolved at this temperature. In a dissolved condition, the preparation of the invention may easily be sterilized by conventional methods, e.g. filtration or by irradiation.

In another aspect, the invention provides a method of preparing a lyophilized preparation comprising the steps of:
(i) providing a preparation as described above in a lyophilization apparatus,
(ii) subjecting the preparation from step (i) to a temperature of -10°C to - 60°C for a predetermined period of time,
(iii) raising the temperature to -10°C to 50°C within a predetermined period of time,
(iv) optionally holding the temperature in step (iii) for a predetermined period of time, and
(v) optionally repeating steps (iii) and (iv) at a higher temperature as compared to that in step (iii) and (iv).

Lyophilization or freeze drying, which may be used interchangeably, are processes in which the solvent is removed from the liquid preparation after it is frozen and placed under reduced pressure, allowing the solvent to sublime. Conventional freeze dryers may be used and are commercially available, for example, at Hof Sonderanlagenbau, Lohra, Germany.

In step (i), the preparation is preferably in the form of a solution.

Steps (ii) and/or (iii) (also including steps (iv) and/or (v)) may be at least partially carried out at a reduced pressure, preferably at 0.001 - 0.2 mbar, more preferably at 0.01-0.1 mbar.

In a preferred embodiment, the predetermined period of time in step (ii) is from 2-6 hours. In another preferred embodiment, the predetermined period of time in step (iv) is from 50-70 hours.

For the lyophilization process, the preparation according to the invention may be directly carried out in suitable containers such as (sterile) vials. When using sterile preparations and sterile vials as described above during the lyophilization process, the method of the present invention provides a streamlined production of a sterile lyophilized preparation.

In a preferred embodiment, the lyophilization cycle may be performed as follows:
(i) The preparation is loaded to a respective lyophilization apparatus at normal pressures.
(ii) The preparation from step (i) is cooled to -40°C to -50°C within 40-80 minutes.
(iii) The temperature is held at -40°C to -50°C for 2-3 hours.
(iv) The pressure is reduced to 0.02-0.06 mbar within 15-40 minutes.
(v) The temperature is increased to -5 to -15°C within 40-80 minutes and held at this temperature for 30-40 hours at a reduced pressure of 0.02-0.06 mbar.
(vi) The temperature is raised to 35-45°C within 6-10 hours and held at this temperature for 15-30 hours at a reduced pressure of 0.02-0.06 mbar.
(vii) The obtained powder is cooled down to room temperature and the reaction vessel is vented, e.g., with an inert gas.

In the above process step (ii) may be regarded as the "freezing phase", step (v) as a "primary drying" (sublimation) and step (vi) as a "secondary drying" (desorption).

The lyophilization process of the present invention removes the liquid medium of the preparation without excess of heating of the product. Moreover, the combination of organic solvent and water prevents an excess hydrolysis of the API. In the lyophilization process, the content of water may be reduced to such an extent that no significant degradation processes such as hydrolysis occurs in the obtained dry powders during storage. Accordingly the above process using the preparations as described above provides lyophilized products exhibiting an overall reduced amount of impurities. Moreover, it has been observed that the dry powders obtained by the lyophilization process of the invention are easily and completely dissolvable in respective reconstitution media.

In one aspect the present invention thus provides a lyophilized preparation obtainable by the process as described above.

Particularly, it has been found that the lyophilized preparation obtainable by the above process contains less than or equal to 2.0 % by weight, preferably ≤ 1.0 % by weight of at least one organic solvent based on the total amount of the preparation and less than or equal to 2.0 % by weight, preferably ≤ 1.5 % by weight of free water based on the total amount of the preparation.

In another aspect, the lyophilized pharmaceutical preparation obtained by the above described process comprises less than or equal to 0.7 % by weight, preferably less than or equal to 0.6 % by weight, even more preferably less than or equal to 0.4 % by weight of API degradation products based on the initial amount of API directly after lyophilization.

In another aspect of the invention, the lyophilized preparation according to the above described process comprises less than or equal to 2.0 % by weight, preferably less than or equal to 1.6 % by weight, more preferably less than or equal to 1.5 % by weight of API degradation products based on the initial amount of API after storage at 40°C for 90 days after lyophilization.

Moreover, it has been found that the such obtained lyophilized preparation can typically comprise less than or equal to 2.0 % by weight, preferably less than or equal to 1.6 % by weight, more preferably less than or equal to 1.5 % by weight of API degradation products based on the initial amount of API after storage for about 6 months, preferably more than about 2 years or preferably more than about 3 years when stored in a closed container at about 20°C to about 25°C.

In case of lyophilized Bendamustine preparations, the API degradation products may be 4-{5-[(2-Chloroethyl)-(2-hydroxy-ethyl)-amino]-1-methyl-1H-benzimidazol-2-yl}-butyric acid (HP1; Monohydroxybendamustine), 4-{5-[bis-(2-hydroxy-ethyl)-amino]-1-methyl-1H-benzimidazol-2-yl}-butyric acid (HP2; Dihydroxybendamustine), 4-(5-morpholino-1-methylbenzimidazol-2-yl)-butanoic acid, 4-{5-Bis-(2-Chloroethyl)-amino]-1-methyl-1H-benzimidazol-2-yl}-butyric acid methylester, 4-{5-Bis-(2-Chloroethyl)-amino]-1-methyl-1H-benzimidazol-2-yl}-butyric acid ethylester, 4-{5-Bis-(2-Chloroethyl)-amino]-1-methyl-1H-benzimidazol-2-yl}-butyric acid isopropylester, 4-[5-((2-((4-(5-(Bis(2-chloroethyl)amino)-1-methyl-1 H-benzo[d]imidazol-2-yl)butanoyl)oxy)ethyl)(2-chloroethyl)amino)-1-methyl-1 H-benzo[d]imidazol-2-yl)butanic acid (Bendamustine Dimer) or mixtures thereof.

In another aspect of the present invention, the lyophilized Bendamustine preparation preferably comprises less than or equal to 0.5 % by weight, preferably less than or equal to 0.3 % by weight, more preferably less than or equal to 0.1 % by weight of HP1, HP2, Bendamustine dimer or mixtures thereof directly after lyophilization.

It has been shown that the lyophilized preparation obtainable by the above-described process is completely reconstitutable in reconstitution media. Suitable reconstitution media may be e.g. distilled (sterile) water, 0.9 % sodium chloride aqueous (sterile) solution, 0.45 % sodium chloride aqueous (sterile) solution, lactated Ringer's injection, glucose 5 % (sterile) solution and mixtures thereof.

The lyophilized preparation can be solved in water at 20°C in less than or equal to 3 minutes, preferably in less than or equal to 60 seconds, even more preferably in less than or equal to 30 seconds with mechanical stirring.

In another aspect, the present invention provides a lyophilized pharmaceutical preparation comprising:
(i) at least one API, a salt, hydrate, solvate or a prodrug thereof,
(ii) at least one bulking agent selected from monosaccharides, disaccharides, oligosaccharides, polysaccharides and sugar alcohols,
(iii) less than or equal to 2.0 % by weight, preferably ≤ 1.0 % by weight of at least one organic solvent based on the total amount of the preparation, and
(iv) less than or equal to 2.0 % by weight, preferably ≤ 1.5 % by weight of free water based on the total amount of the preparation.

The API used in the lyophilized pharmaceutical preparation may be the same as described above.

The bulking agent used in the lyophilized pharmaceutical preparation may be the same as described above.

The organic solvent may be particularly an organic solvent which is pharmaceutically acceptable and more preferably has a vapor pressure of 1.0 to 41 at 20°C. The organic solvent may be same as used in the preparation of the invention. Traces of organic solvent, such as 0.001% by weight or 0.01% by weight based on the total amount of the preparation may be present in the pharmaceutical preparation.

Traces of water, such as 0.001% by weight or 0.01% by weight or 2.0% by weight based on the total amount of the preparation may be present in the pharmaceutical preparation.

In a preferred embodiment, the lyophilized pharmaceutical preparation comprises 10-80 % by weight, more preferably 10-60 % by weight, more preferably 20-50 % by weight of API based on the total amount of the preparation.

In another aspect of the invention, the lyophilized pharmaceutical preparation comprises 20-90 % by weight, preferably 40-90 % by weight, more preferably 50-80 % by weight of at least one bulking agent based on the total amount of the preparation.

The lyophilized pharmaceutical preparation may further comprise pharmaceutically acceptable excipients, such as stabilizers, buffers, solubilizers, anti-bacterial agents such as paraben, chlorobutanol, phenol, ascorbic acid and dispersion medium such as polyose (e.g. glycerol, propylenglycol, liquid polyethylene glycol), fats (e.g. vegetable oils, non-toxic glycerol esters) and mixtures thereof.

The lyophilized pharmaceutical preparation may further comprise a further pharmaceutical active agent such as, for example, another antineoplastic agent.

In a preferred embodiment, the lyophilized pharmaceutical preparation comprises less than or equal to 0.7 % by weight, preferably less than or equal to 0.6 % by weight, even more preferably less than or equal to 0.4 % by weight of API degradation products based on the initial amount of API directly after lyophilization. Traces of API degradation products, such as 0.001% by weight or 0.01% by weight based on the initial amount of API may be present in the lyophilized pharmaceutical preparation.

In another embodiment, the lyophilized pharmaceutical preparation comprises less than or equal to 2.0 % by weight, preferably less than or equal to 1.6 % by weight, more preferably less than or equal to 1.5 % by weight of API degradation products based on the initial amount of API after storage at 40°C for 90 days after lyophilization.

In lyophilized pharmaceutical Bendamustine preparations according to the invention, the API degradation product may be selected from 4-{5-[(2-Chloroethyl)-(2-hydroxy-ethyl)-amino]-1-methyl-1H-benzimidazol-2-yl}-butyric acid (HP1; Monohydroxybendamustine), 4{5-[bis-(2-hydroxy-ethyl)-amino]-1-methyl-1H-benzimidazol-2-yl}-butyric acid (HP2; Dihydroxybendamustine), 4-(5-morpholino-1-methylbenzimidazol-2-yl)-butanoic acid , 4-{5-Bis-(2-Chloroethyl)-amino]-1-methyl-1H-benzimidazol-2-yl}-butyric acid methylester, 4-{5-Bis-(2-Chloroethyl)-amino]-1-methyl-1H-benzimidazol-2-yl}-butyric acid ethylester, 4-{5-Bis-(2-Chloroethyl)-amino]-1-methyl-1H-benzimidazol-2-yl}-butyric acid isopropylester, 4-[5-((2-((4-(5-(Bis(2-chloroethyl)amino)-1-methyl-1H-benzo[d]imidazol-2-yl)butanoyl)oxy)ethyl)(2-chloroethyl)amino)-1-methyl-1H-benzo[d]imidazol-2-yl)butanic acid (Bendamustine Dimer) or mixtures thereof.

According to a preferred embodiment the lyophilized pharmaceutical Bendamustine preparation comprises less than or equal to 0.5 % by weight, preferably less than or equal to 0.3 % by weight, more preferably less than or equal to 0.1 % by weight of HP1, HP2, Bendamustine dimer or mixtures thereof directly after lyophilization.

The lyophilized pharmaceutical preparation of the present invention can be preferably solved in water at 20°C in less than or equal to 3 minutes, preferably in less than or equal to 60 seconds, even more preferably in less than or equal to 30 seconds with mechanical stirring.

The present invention further provides a container comprising a lyophilized preparation obtainable by the process described above or a lyophilized pharmaceutical preparation as described above containing a total amount of API or a salt, hydrate, solvent or prodrug thereof of 1-500 mg, more preferably 1-300 mg, even more preferably 2-200 mg, and most preferably 2-150 mg.

The container may be made from rigid or flexible material such as glass or polymeric materials. The container is preferably a vial as used in pharmaceutical applications. The container of the present invention may be the same container which was used in the lyophilization process for obtaining the pharmaceutical preparation. The container may be configured such that reconstitution with a reconstitution medium may be carried out directly within the container.

In a preferred embodiment, the container comprises a lyophilized Bendamustine pharmaceutical preparation, preferably comprising about 10-500 mg, preferably 25-100 mg, of Bendamustine or a salt thereof, and preferably 10-500 mg (more preferably 25-100 mg) of a bulking agent.

Another aspect of the present invention is a reconstituted pharmaceutical preparation comprising a lyophilized preparation obtained according to the process described above or a lyophilized pharmaceutical preparation as described above and at least one reconstitution medium. Suitable reconstitution media may be distilled (sterile) water, 0.9 % sodium chloride aqueous (sterile) solution, 0.45 % sodium chloride aqueous (sterile) solution, lactated Ringer's injection, glucose 5 % (sterile) solution and mixtures thereof.

Preferably, the reconstituted pharmaceutical preparation exhibits a pH value in the range of 3-8. This pH range allows a limited degradation such as hydrolysis of the API. The thus obtained reconstituted preparations may be used in pharmaceutical therapy, particularly for parenteral such as intravenous, intra-arterial, intra-muscular, subcutaneous and intra-peritoneal administration.

Reconstituted pharmaceutical preparation comprising nitrogen mustards, such as Bendamustine as an API, may be used for treating medical conditions selected from chronic lymphocytic leukemia, acute lymphocytic leukemia, chronic myelocytic leukemia, acute myelocytic leukemia, Hodgkin's disease, non-Hodgkin's lymphoma, multiple myeloma, breast cancer, ovarian cancer, small-cell lung cancer, non-small-cell lung cancer and autoimmune diseases in a human or animal body comprising administering to the human or animal body in need thereof an effective amount of the nitrogen mustard. In case of a reconstituted pharmaceutical bendamustine preparations preferably the medical condition is non-Hodgkin's lymphoma.

Reconstituted pharmaceutical preparations comprising cytostatic agents such as Fludarabine, Doxorubicine, Pemetrexed and Dacarbazine as an API may be used for treating medical conditions selected from hyperproliferative disorders such as cancer, particularly in a human or animal body comprising administering to the human or animal body in need thereof an effective amount of the cytostatic agent.

### Examples

### 1. Materials and Apparatus

### Chemicals:

Bendamustine HCl x 1 H₂O (NerPharMa DS Srl), D(-)-Mannitol ≥98%; Ph. Eur. (Carl Roth), DL-1,2-isopropylideneglycerol (Solketal) 98% (Sigma Aldrich), 1,4-Dioxane ≥99% (Sigma Aldrich), (-)-Ethyl L-lactate ≥98.0% (Sigma Aldrich), Acetonitrile (Merck), Trifluoroacetic acid (TFA) (Merck), Dimethyl sulfoxide (DMSO) ≥99.8% (Carl Roth).

### Freeze dryer:

- Manufacturer: Hof Sonderanlagenbau (Lohra, Germany)
- 0.36 m² shelf area
- Differential pressure measurement

### HPLC:

- HPLC unit: 1100 series
- Manufacturer: Agilent (Santa Clara, USA)
   - Degasser: G1322A
   - BinPump: G1312A
   - Auto Sampler: G1329A
   - Auto Sampler Thermostat: G1330B
   - Column Oven: G1316A
   - DAD: G1315A

### Gas chromatograph:

- Type: HP 6890A / Agilent 7694 Headspace sampler
- Manufacturer: Hewlett Packard /Agilent (Santa Clara, USA)

### 2. Measurements

### RP-HPLC:

The purity of API was determined by RP-HPLC. DMSO was used for dilution of the lyophilizates. The autosampler temperature was set to 25°C to avoid crystallization of DMSO (melting point at 18°C). The dilutions have been prepared directly after the lyophilisation process (if not indicated differently) and have been directly measured by RP-HPLC under the following conditions:

| | |
|---|---|
| Column type: | Kinetex C18, 100 x 4.6 mm, 2.6 µm (Phenomenex) |
| Mobile phase: | A: Water/ACN/TFA = 950/50/1 v/v/v |
| | B: Water/ACN/TFA = 50/950/1 v/v/v |
| Column temperature: | 25°C |
| Autosampler temperature: | 25°C (5°C original value) |
| Flow rate: | 1.2mL/min |
| Wavelength detection: | 234 nm |
| Slit: | 4 nm |
| Reference wavelength: | 550 nm |
| Reference slit: | 100 nm |
| Detection range: | 0-30 min |

### HPLC Gradient

| **Time** | **Eluent A** | **Eluent B** |
|---|---|---|
| **[min]** | | |
| 0 | 95% | 5% |
| 5 | 95% | 5% |
| 9 | 80% | 20% |
| 25 | 55% | 45% |
| 28 | 20% | 80% |
| 30 | 20% | 80% |
| 30.01 | 95% | 5% |
| 35 | 95% | 5% |

### Gas Chromatography - Residual Water and Solvent Measurements

Water is determined after the reaction of 2,2-dimethoxypropane by measuring the quantity of formed acetone.

Sample preparation:
- Prepare a reaction solution containing 0.16 ml/ml 2,2-dimethoxypropane and 2.4 µl/ml methansulfonic acid in DMSO.
- Dissolve the sample in an appropriate amount of a 1/1 mixture of reaction solution and DMSO.

Preparation of standards:
- Prepare a stock solution containing 1% water in DMSO. Other standards can be prepared additionally.
- Prepare standards according to the following matrix in one GC Vial. Standards are prepared in duplicates.

| **Water content** | **Reaction solution** | **Water stock solution** | **DMSO** | |
|---|---|---|---|---|
| | **Mg** | **mL** | **mL** | **mL** |
| No addition of water | +0 | 1.0 | - | 1.0 |
| 0.05% water | +1 | 1.0 | 0.1 | 0.9 |
| 0.2% water | +4 | 1.0 | 0.4 | 0.6 |
| 0.5% water | +10 | 1.0 | 1.0 | - |

DMSO is hygroscopic. For that reason the calibration curve was corrected by the initial residual water content of the utilized DMSO.

The quantification of residual solvents with the GC method is used for the determination, identification and control of residual solvents (see *EUROPEAN PHARMACOPOEIA* 5.0. 2.4.24.). The solvents used for dilution were: water for 1,4-dioxane lyophilizates and DMSO for ethyl lactate lyophilizates. The calibration standards of the two solvents were performed in the range of 0 - 10 mg. The resulting measurement range went from 0% to 13.5% residual organic solvent for a lyophilizate weight of 72 mg.

### 3. Manufacture of the Preparation

Samples were manufactured of each variant. The compositions are listed in the following table:

| | **1,4-Dioxane** | **Ethyl lactate** |
|---|---|---|
| **Solvent** | 40% | 40% |
| **Water** | 60% | 60% |
| | | |

| | **[mg]** | **[mg]** |
|---|---|---|
| Bendamustine HCl x 1 H₂O | 25.0 | 25.0 |
| Mannitol | 47.0 | 47.0 |
| Solvent | 371.2 | 371.2 |
| Water | 556.8 | 556.8 |

Bendamustine hydrochloride monohydrate has been dissolved together with Mannitol in the solvent/water mixture resulting in preparation for the vial filling.

### 4. Lyophilization

The respective vials were filled with the 1,000 mg preparations as prepared under item 3. Lyophilisation was carried out in stainless steel racks sealed into lyo-bags to avoid contamination of the freeze dryer. The prototype lyophilisation cycle is indicated in the below Table.

### Lyophilization parameters

| **Step** | **Step duration** | **Total duration** | **Shelf temperature** | **Condenser temperature** | **Pressure** |
|---|---|---|---|---|---|
| | [hh:mm] | [hh:mm] | [°C] | [°C] | [mbar] |
| Loading | 00:30 | 0:30 | 20 | 0 | 1000 |
| Freezing | 01:00 | 1:30 | -45 | 0 | 1000 |
| Freezing | 02:30 | 4:00 | -45 | 0 | 1000 |
| Vacuum Adjustment | 00:30 | 4:30 | -45 | -80 | 0.05 |
| Primary Drying | 01:00 | 5:30 | -10 | -80 | 0.05 |
| Primary Drying | 35:00 | 40:30 | -10 | -80 | 0.05 |
| Secondary Drying | 08:00 | 48:30 | 40 | -80 | 0.05 |
| Secondary Drying | 20:00 | 68:30 | 40 | -80 | 0.05 |

After completion of the lyophilization, the drying chamber was vented to 800 mbar with nitrogen and closed by collapsing the shelves.

### 5. Results

### Visual Examination

The lyophilizate cakes in the vial obtained with 1,4-dioxane and ethyl lactate had an excellent visual appearance without defects.

### RP-HPLC Measurements - Impurities

Two examples of each variant were analysed for respective impurities. The results are shown in the below Table.

**Table: Results pf RP-HPLC**

| **Time** | **Sample** | **Purity Bendamustine Hydrochloride** | **Hydrolysis** | **Dimer product** | **Unknown** |
|---|---|---|---|---|---|
| After lyophilization | 1,4-Dioxane vial 1 | 99.8% | 0.1% | | 0.1% |
| | 1,4-Dioxane vial 2 | 99.8% | 0.1% | | 0.1% |
| | Ethyl lactate vial 1 | 99.8% | 0.1% | | 0.1% |
| | Ethyl lactate vial 2 | 99.8% | 0.1% | | 0.1% |
| | Solketal vial 1 | 99.3% | 0.2% | 0.1% | 0.4% |
| | Solketal vial 2 | 99.3% | 0.2% | 0.1% | 0.4% |
| After 30 days storage at 30°C | 1,4-Dioxane vial 1 | 99.4% | 0.1% | 0.1% | 0.4% |
| | 1,4-Dioxane vial 2 | 99.4% | 0.1% | 0.1% | 0.4% |
| | Ethyl lactate vial 1 | 99.4% | 0.1% | 0.1% | 0.3% |
| | Ethyl lactate vial 2 | 99.5% | 0.1% | 0.1% | 0.3% |
| After 30 days storage at 40°C | 1,4-Dioxane vial 1 | 99.0% | 0.2% | 0.2% | 0.6% |
| | 1,4-Dioxane vial 2 | 99.0% | 0.2% | 0.2% | 0.6% |
| | Ethyl lactate vial 1 | 98.9% | 0.2% | 0.2% | 0.6% |
| | Ethyl lactate vial 2 | 99.0% | 0.2% | 0.2% | 0.6% |
| After 60 days storage at 30°C | 1,4-Dioxane vial 1 | 99.3% | 0.1% | 0.1% | 0.4% |
| | 1,4-Dioxane vial 2 | 99.3% | 0.1% | 0.2% | 0.4% |
| | Ethyl lactate vial 1 | 99.3% | 0.2% | 0.2% | 0.4% |
| | Ethyl lactate vial 2 | 99.2% | 0.2% | 0.2% | 0.4% |
| After 60 days storage at 40°C | 1,4-Dioxane vial 1 | 98.6% | 0.2% | 0.3% | 0.8% |
| | 1,4-Dioxane vial 2 | 98.6% | 0.2% | 0.3% | 0.8% |
| | Ethyl lactate vial 1 | 98.4% | 0.2% | 0.3% | 1.0% |
| | Ethyl lactate vial 2 | 98.4% | 0.3% | 0.3% | 1.0% |
| After 90 days storage at 40°C | 1,4-Dioxane vial 1 | 98.4% | 0.2% | 0.3% | 1.0% |
| | 1,4-Dioxane vial 2 | 98.4% | 0.2% | 0.3% | 1.0% |
| | Ethyl lactate vial 1 | 98.5% | 0.3% | 0.3% | 1.0% |
| | Ethyl lactate vial 2 | 98.4% | 0.2% | 0.3% | 1.0% |

### Gas Chromatography - Residual Water and Solvent Measurements

Gas chromatography results are shown in the below Table. The organic solvents were below the detection limit of 0.4 mg. With a lyophilizate weight of 72 mg, this value represents a residual organic solvent content less than 0.5 %. The residual water was about 2 % in all variants, but assuming Bendamustine hydrochloride being present as monohydrate, the free water values drops to a very low level of about 0.3-0.4 %.

**Table: Measured residual water and organic solvents per vial**

| **Sample** | **Measured residual organic solvent** | **Measured residual water** |
|---|---|---|
| | **mg** | **mg** |
| 1,4-Dioxane vial 1 | <0.4 | 1.4* |
| 1,4-Dioxane vial 2 | <0.4 | 1.5* |
| Ethyl lactate vial 1 | <0.4 | 1.4* |
| Ethyl lactate vial 2 | <0.4 | 1.5* |

| | | |
|---|---|---|
| *It has to be taken into account that if Bendamustine Hydrochloride occurs as monohydrate 1.4 mg measured water correspond to 0.2 mg free water and 1.5 mg measured water correspond to 0.3 mg free water. | | |

The following items are subject-matter of the present invention:
1. Preparation comprising:
   (i) at least one active pharmaceutical ingredient (API), a salt, hydrate, solvate or prodrug thereof,
   (ii) at least one bulking agent selected from monosaccharides, disaccharides, oligosaccharides, polysaccharides and sugar alcohols,
   (iii) at least one organic solvent having a vapor pressure of 1.0 to 41 mbar at 20°C, and
   (iv) water.
2. Preparation according to item 1, wherein the preparation is a solution at room temperature.
3. Preparation according to any one of items 1 or 2, wherein the API is not stable in the presence of water or humidity.
4. Preparation according to any one of items 1-3, wherein the API is a nitrogen mustard such as Bendamustine, Busulfan, Melphalan, Uramustine, Ifosfamide, Chlorambucil and Cyclophosphamide, cytostatic agents such as Fludarabine, Doxorubicine, Pemetrexed and Dacarbazine as well as an opioid such as Remifentanil, particularly preferred is Bendamustine.
5. Preparation according to any one of items 1-4, wherein the bulking agent is selected from mannitol, maltitol, erythritol, xylitol, lactose, sucrose, glucose, sorbitol, maltose, trehalose, lactitol and dextrose, preferably mannitol.
6. Preparation according to any one of items 1-5, wherein the organic solvent is a pharmaceutically acceptable solvent.
7. Preparation according to any one of items 1-6, wherein the organic solvent is selected from ethyl lactate, dioxane, isopropylidene glycerol (solketal) acetic acid, acetyl acetone, 1-butanol, 1-propanol, N,N-dimethylacetamide, cyclohexanone, and toluene, particularly ethyl lactate and dioxane, more preferably ethyl lactate.
8. Preparation according to any one of items 1-7 which comprises 1 to 80 % by weight, preferably 20 to 60 % by weight, even more preferably 30 to 50 % by weight of at least one organic solvent based on the total amount of the preparation.
9. Preparation according to any one of items 1-8 which comprises 19.3 to 99.3 % by weight, preferably 20 to 95 % by weight, more preferably 40 to 75 % by weight, even more preferably 40 to 70 % by weight of water based on the total amount of the preparation.
10. Preparation according to any one of items 1-9 which comprises 0.2 to 10 % by weight, more preferably 0.5 to 5 % by weight, even more preferably 0.5 to 4 % by weight of at least one API based on the total amount of the preparation.
11. Preparation according to any one of items 1-10 which comprises 0.5 to 15 % by weight, preferably 0.5 to 10 % by weight, more preferably 1 to 7 % by weight of at least one bulking agent based on the total amount of the preparation.
12. Method of preparing a lyophilized preparation comprising the steps of:
   (i) providing a preparation according to any one of items 1-11 in a lyophilization apparatus,
   (ii) subjecting the preparation from step (i) to a temperature of -10°C to - 60°C for a predetermined period of time,
   (iii) raising the temperature to -10°C to 50°C within a predetermined period of time,
   (iv) optionally holding the temperature in step (iii) for a predetermined period of time, and
   (v) optionally repeating steps (iii) and (iv) at a higher temperature as compared to that in steps (iii) and (iv).
13. Method according to item 12, wherein step (ii) and/or step (iii) is at least partially carried out at an reduced pressure, preferably at 0.001 to 0.2 mbar.
14. Method according to any one of items 12 or 13, wherein the predetermined period of time in step (ii) is from 2 to 6 hours.
15. Method according to any one of items 12-14, wherein the predetermined period of time in step (iv) is from 50 to 70 hours.
16. Lyophilized preparation obtainable by a process according to any one of items 12-15.
17. Lyophilized pharmaceutical preparation comprising:
   (i) at least one API, a salt, hydrate, solvate or prodrug thereof,
   (ii) at least one bulking agent selected from monosaccharides, disaccharides, oligosaccharides, polysaccharides and sugar alcohols,
   (iii) less than or equal to 2.0 % by weight, preferably ≤ 1.0 % by weight of at least one organic solvent based on the total amount of the preparation, and
   (iv) less than or equal to 2.0 % by weight, preferably ≤ 1.5 % by weight of free water based on the total amount of the preparation.
18. Lyophilized pharmaceutical preparation according to item 17, wherein the API is not stable in the presence of water or humidity.
19. Lyophilized pharmaceutical preparation according to any one of items 17 or 18, wherein the API is a nitrogen mustard such as Bendamustine, Busulfan, Melphalan, Uramustine, Ifosfamide, Chlorambucil and Cyclophosphamide, cytostatic agents such as Fludarabine, Doxorubicine, Pemetrexed and Dacarbazine as well as an opioid such as Remifentanil, particularly preferred is Bendamustine.
20. Lyophilized pharmaceutical preparation according to any one of items 17-19, wherein the bulking agent is selected from mannitol, maltitol, erythritol, xylitol, lactose, sucrose, glucose, sorbitol, maltose, trehalose, lactitol and dextrose, preferably mannitol.
21. Lyophilized pharmaceutical preparation according to any one of items 17-20, wherein the organic solvent is a pharmaceutically acceptable solvent, particularly having a vapor pressure of 1.0 to 41 mbar at 20°C.
22. Lyophilized pharmaceutical preparation according to any one of items 17-21, wherein the organic solvent is selected from ethyl lactate, dioxane, isopropylidene glycerol (solketal), acetic acid, acetyl acetone, 1-butanol, 1-propanol, N,N-dimethylacetamide, cyclohexanone, and toluene, particularly from ethyl lactate and dioxane, more preferably from ethyl lactate.
23. Lyophilized pharmaceutical preparation according to any one of items 17-22 which comprises 10 to 80 % by weight, preferably 10 to 60 % by weight, more preferably 20 to 50 % by weight of API based on the total amount of the preparation.
24. Lyophilized pharmaceutical preparation according to any one of items 17-23 which comprises 20 to 90 % by weight, preferably 40 to 90 % by weight, more preferably 50 to 80 % by weight of at least one bulking agent based on the total amount of the preparation.
25. Lyophilized pharmaceutical preparation according to any one of items 17-24, comprising less than or equal to 0.7 % by weight, preferably less than or equal to 0.6 % by weight, even more preferably less than or equal to 0.4 % by weight of API degradation products based on the initial amount of API directly after lyophilization.
26. Lyophilized pharmaceutical preparation according to any one of items 17-25 comprising less than or equal to 2.0 % by weight, preferably less than or equal to 1.6 % by weight, more preferably less than or equal to 1.5 % by weight of API degradation products based on the initial amount of API after storage at 40°C for 90 days after lyophilization.
27. Lyophilized pharmaceutical preparation according to any one of items 19-26, wherein the API degradation product is 4-{5-[(2-Chloroethyl)-(2-hydroxy-ethyl)-amino]-1-methyl-1H-benzimidazol-2-yl}-butyric acid (HP1), 4{5-[bis-(2-hydroxy-ethyl)-amino]-1-methyl-1H-benzimidazol-2-yl}-butyric acid (HP2), 4-(5-morpholino-1-methylbenzimidazol-2-yl)-butanoic acid , 4-{5-Bis-(2-Chloroethyl)-amino]-1-methyl-1H-benzimidazol-2-yl}-butyric acid methylester, 4-{5-Bis-(2-Chloroethyl)-amino]-1-methyl-1H-benzimidazol-2-yl}-butyric acid ethylester, 4-{5-Bis-(2-Chloroethyl)-amino]-1-methyl-1H-benzimidazol-2-yl}-butyric acid isopropylester, 4-[5-((2-((4-(5-(Bis(2-chloroethyl)amino)-1-methyl-1H-benzo[d]imidazol-2-yl)butanoyl)oxy)ethyl)(2-chloroethyl)amino)-1-methyl-1 H-benzo[d]imidazol-2-yl)butanic acid (Bendamustine Dimer) or mixtures thereof.
28. Lyophilized pharmaceutical preparation according to any one of items 17-27 which comprises less than or equal to 0.5 % by weight, preferably less than or equal to 0.3 % by weight, more preferably less than or equal to 0.1 % by weight of HP1, HP2, Bendamustine dimer or mixtures thereof directly after lyophilization.
29. Lyophilized pharmaceutical preparation according to any one of items 17-28 which is solved in water at 20°C in less than or equal to 3 minutes, preferably in less than or equal to 60 seconds, even more preferably in less than or equal to 30 seconds with mechanical stirring.
30. Container comprising a lyophilized pharmaceutical preparation according to any one of items 17-29 containing a total amount of API, a salt, hydrate, solvate or prodrug thereof of 1 to 500 mg, more preferably 1 to 300 mg, even more preferably 2 to 200 mg, most preferably 2 to 150 mg.
31. Reconstituted pharmaceutical preparation comprising:
   (i) a lyophilized preparation according to item 16 or a lyophilized preparation according to any of items 17-29, and
   (ii) at least one reconstitution medium.
32. Reconstituted pharmaceutical preparation according to item 31, wherein the reconstitution medium is selected from distilled (sterile) water, 0.9 % sodium chloride aqueous (sterile) solution, 0.45 % sodium chloride aqueous (sterile) solution, lactated Ringer's injection, glucose 5 % (sterile) solution and mixtures thereof.
33. Reconstituted pharmaceutical preparation according to item 31 or 32 for use for treating medical conditions selected from chronic lymphocytic leukemia, acute lymphocytic leukemia, chronic myelocytic leukemia, acute myelocytic leukemia, Hodgkin's disease, non-Hodgkin's lymphoma, multiple myeloma, breast cancer, ovarian cancer, small cell lung cancer, non-small cell lung cancer and autoimmune diseases in a human or animal body.

## Claims

1. Preparation comprising:
(i) at least one active pharmaceutical ingredient (API), a salt, hydrate, solvate or prodrug thereof,
(ii) at least one bulking agent selected from monosaccharides, disaccharides, oligosaccharides, polysaccharides and sugar alcohols,
(iii) at least one organic solvent having a vapor pressure of 1.0 to 41 mbar at 20°C, and
(iv) water.

2. Preparation according to claim 1, wherein the API is a nitrogen mustard such as Bendamustine, Busulfan, Melphalan, Uramustine, Ifosfamide, Chlorambucil and Cyclophosphamide, cytostatic agents such as Fludarabine, Doxorubicine, Pemetrexed and Dacarbazine as well as an opioid such as Remifentanil, particularly preferred is Bendamustine.

3. Preparation according to any one of claims 1-2, wherein the bulking agent is selected from mannitol, maltitol, erythritol, xylitol, lactose, sucrose, glucose, sorbitol, maltose, trehalose, lactitol and dextrose, preferably mannitol.

4. Preparation according to any one of claims 1-3, wherein the organic solvent is selected from ethyl lactate, dioxane, isopropylidene glycerol (solketal), acetic acid, acetyl acetone, 1-butanol, 1-propanol, N,N-dimethylacetamide, cyclohexanone, and toluene, particularly ethyl lactate and dioxane, more preferably ethyl lactate.

5. Method of preparing a lyophilized preparation comprising the steps of:
(i) providing a preparation according to any one of claims 1-4 in a lyophilization apparatus,
(ii) subjecting the preparation from step (i) to a temperature of -10°C to - 60°C for a predetermined period of time,
(iii) raising the temperature to -10°C to 50°C within a predetermined period of time,
(iv) optionally holding the temperature in step (iii) for a predetermined period of time, and
(v) optionally repeating steps (iii) and (iv) at a higher temperature as compared to that in steps (iii) and (iv).

6. Method according to claim 5, wherein step (ii) and/or step (iii) is at least partially carried out at an reduced pressure, preferably at 0.001 to 0.2 mbar.

7. Lyophilized preparation obtainable by a process according to any one of claims 5 or 6.

8. Lyophilized pharmaceutical preparation comprising:
(i) at least one API, a salt, hydrate, solvate or prodrug thereof,
(ii) at least one bulking agent selected from monosaccharides, disaccharides, oligosaccharides, polysaccharides and sugar alcohols,
(iii) less than or equal to 2.0 % by weight, preferably ≤ 1.0 % by weight of at least one organic solvent based on the total amount of the preparation, and
(iv) less than or equal to 2.0 % by weight, preferably ≤ 1.5 % by weight of free water based on the total amount of the preparation.

9. Lyophilized pharmaceutical preparation according to claim 8, wherein the API is a nitrogen mustard such as Bendamustine, Busulfan, Melphalan, Uramustine, Ifosfamide, Chlorambucil and Cyclophosphamide, cytostatic agents such as Fludarabine, Doxorubicine, Pemetrexed and Dacarbazine as well as an opioid such as Remifentanil, particularly preferred is Bendamustine.

10. Lyophilized pharmaceutical preparation according to any one of claims 8 or 9, wherein the bulking agent is selected from mannitol, maltitol, erythritol, xylitol, lactose, sucrose, glucose, sorbitol, maltose, trehalose, lactitol and dextrose, preferably mannitol.

11. Lyophilized pharmaceutical preparation according to any one of claims 8-10, wherein the organic solvent is selected from ethyl lactate, dioxane, acetic acid, isopropylidene glycerol (solketal), acetyl acetone, 1-butanol, 1-propanol, N,N-dimethylacetamide, cyclohexanone, and toluene, particularly from ethyl lactate and dioxane, more preferably from ethyl lactate.

12. Lyophilized pharmaceutical preparation according to any one of claims 8-11, comprising less than or equal to 0.7 % by weight, preferably less than or equal to 0.6 % by weight, even more preferably less than or equal to 0.4 % by weight of API degradation products based on the initial amount of API directly after lyophilization.

13. Lyophilized pharmaceutical preparation according to any one of claims 8-12, wherein the API degradation product is 4-{5-[(2-Chloroethyl)-(2-hydroxy-ethyl)-amino]-1-methyl-1H-benzimidazol-2-yl}-butyric acid (HP-1), 4{5-[bis-(2-hydroxy-ethyl)-amino]-1-methyl-1H-benzimidazol-2-yl}-butyric acid (HP-2), 4-(5-morpholino-1-methylbenzimidazol-2-yl)-butanoic acid, 4-{5-Bis-(2-Chloroethyl)-amino]-1-methyl-1 H-benzimidazol-2-yl}-butyric acid methylester, 4-{5-Bis-(2-Chloroethyl)-amino]-1-methyl-1H-benzimidazol-2-yl}-butyric acid ethylester, 4-{5-Bis-(2-Chloroethyl)-amino]-1-methyl-1H-benzimidazol-2-yl}-butyric acid isopropylester, 4-[5-((2-((4-(5-(Bis(2-chloroethyl)amino)-1-methyl-1 H-benzo[d]imidazol-2-yl)butanoyl)oxy)ethyl)(2-chloroethyl)amino)-1-methyl-1 H-benzo[d]imidazol-2-yl)butanic acid (Bendamustine Dimer) or mixtures thereof.

14. Container comprising a lyophilized pharmaceutical preparation according to any one of claims 7-13 containing a total amount of API, a salt, hydrate, solvate or prodrug thereof of 1 to 500 mg, more preferably 1 to 300 mg, even more preferably 2 to 200 mg, most preferably 2 to 150 mg.

15. Reconstituted pharmaceutical preparation comprising:
(i) a lyophilized preparation according to claim 7 or a lyophilized preparation according to any of claims 8-13, and
(ii) at least one reconstitution medium.
